# EUROPEAN PATENT APPLICATION

(11) **EP 3 231 384 A1**
(43) Date of publication of application: **18.10.2017**
(21) Application number: 17166327.1
(22) Date of filing: 12.04.2017
(51) Int. Cl.: A61B 18/14

(54) **PULMONARY-VEIN CORK DEVICE WITH ABLATION GUIDING TRENCH**

(30) Priority: 13.04.2016 US 201615097641
(71) Applicant: Biosense Webster (Israel) Ltd., 2066717 Yokneam (IL)
(72) Inventor: BOTZER, Lior, 2066717 Yokneam (IL); SABA, Eitan Moshe, 2066717 Yokneam (IL); RAVUNA, Eliyahu, 2066717 Yokneam (IL); AUERBACH, Shmuel, 2066717 Yokneam (IL); BAR-TAL, Meir, 2066717 Yokneam (IL)
(74) Representative: Carpmaels & Ransford LLP

(57) **Abstract**

A method includes inserting into a patient body a catheter, which includes an insertion tube and at least an expandable distal-end device coupled to a distal end of the insertion tube. The distal-end device is expanded in a blood vessel, thereby forming an annular guiding trench between the distal-end device and a circumference of the blood vessel, the annular trench shaped to receive and guide a distal tip of a medical instrument. The distal tip of the medical instrument is guided in the annular guiding trench formed by the expanded distal-end device of the catheter, so as to approach multiple points on the circumference of the blood vessel.

## Description

### FIELD OF THE INVENTION

The present invention relates generally to ablation of tissue, and specifically to circumferential ablation of a blood vessel.

### BACKGROUND OF THE INVENTION

Ablation techniques are in common use for treating various heart conditions such as cardiac arrhythmia. Examples of prior art techniques are provided below.

U.S. Patent 6,572,612, whose disclosure is incorporated herein by reference, describes a catheter assembly and method for treatment of cardiac arrhythmia. The catheter assembly includes a catheter body and at least one electrode. The catheter body includes a proximal portion, an intermediate portion and a distal portion. The intermediate portion extends from the proximal portion and defines a longitudinal axis. The distal portion extends from the intermediate portion and forms a substantially closed loop transverse to the longitudinal axis. The at least one electrode is disposed along the loop. With this configuration, the loop is axially directed into contact with the chamber wall about the vessel ostium. Upon energization, the electrode ablates a continuous lesion pattern about the vessel ostium, thereby electrically isolating the vessel from the chamber.

U.S. Patent 6,491,710, whose disclosure is incorporated herein by reference, describes a balloon catheter capable of forming a transmural necrotic layer around the pulmonary vein ostium without excessively cauterizing the endocardium and of cauterizing portions of the four pulmonary veins around the ostium of the same one by one. The balloon catheter comprises: a catheter shaft consisting of a tubular outer shaft and a tubular inner shaft, an inflatable balloon capable of coming into contact with a predetermined annular portion of the entrance of a pulmonary vein when inflated, a radio-frequency electrode paired with a counter electrode placed on the surface of a patient's body to transmit radio-frequency power, and placed in a wall forming the balloon or in the balloon, a lead wire electrically connected to the radio-frequency electrode, a cooling means for pouring cooling liquid to cool respective interior of the catheter shaft and the balloon, and a temperature sensor for measuring temperature in the balloon.

Documents incorporated by reference in the present patent application are to be considered an integral part of the application except that, to the extent that any terms are defined in these incorporated documents in a manner that conflicts with definitions made explicitly or implicitly in the present specification, only the definitions in the present specification should be considered.

### SUMMARY OF THE INVENTION

An embodiment of the present invention that is described herein provides a method including inserting into a patient body a catheter, which includes an insertion tube and at least an expandable distal-end device coupled to a distal end of the insertion tube. The distal-end device is expanded in a blood vessel, thereby forming an annular guiding trench between the distal-end device and a circumference of the blood vessel, the annular trench shaped to receive and guide a distal tip of a medical instrument. The distal tip of the medical instrument is guided in the annular guiding trench formed by the expanded distal-end device of the catheter, so as to approach multiple points on the circumference of the blood vessel.

In some embodiments, expanding the distal-end device includes inflating the distal-end device using a saline solution. In other embodiments, the saline solution includes dye visible to X-ray radiation. In yet other embodiments, the distal-end device includes a material that is opaque to X-ray radiation.

In an embodiment, the blood vessel includes a pulmonary vein (PV). In another embodiment, expanding the distal-end device includes inflating the distal-end device so as to form a shape that fits a circumference of an ostium of the blood vessel. In yet another embodiment, the distal-end device includes at least one opening, which is configured to allow blood flow through the distal-end device when the distal-end device is expanded in the blood vessel.

In some embodiments, the distal-end device includes a protrusion, and the annular guiding trench is formed between the protrusion and the circumference of the blood vessel. In other embodiments, the distal tip of the medical instrument includes an ablation electrode, and guiding the distal tip includes ablating multiple points around the circumference of the blood vessel using the ablation electrode. In yet other embodiments, the distal-end device includes multiple foldable arms, and expanding the distal-end device includes unfolding the arms so as to form the annular trench.

In an embodiment, the distal-end device includes an expandable spring, and expanding the distal-end device includes expanding the expandable spring so as to form the annular trench. In another embodiment, expanding the distal-end device includes expanding an anchoring extension in the blood vessel, thereby fixing the distal-end device to the circumference of the blood vessel.

There is additionally provided, in accordance with an embodiment of the present invention, a catheter including an insertion tube for insertion into a patient body and at least an expandable distal-end device coupled to a distal end of the insertion tube. The expandable device is configured to expand the blood vessel thereby forming an annular guiding trench between the distal-end device and a circumference of the blood vessel, the annular trench shaped to receive and guide a distal tip of a medical instrument.

The present disclosure will be more fully understood from the following detailed description of the embodiments thereof, taken together with the drawings, in which:

### BRIEF DESCRIPTION OF THE DRAWINGS

FIG. 1 is a schematic, pictorial illustration of a catheter-based tracking and ablation system, in accordance with an embodiment of the present invention;
FIG. 2 is a schematic side view of a distal end of a catheter, in accordance with an embodiment of the present invention;
FIG. 3 is a schematic, pictorial illustration of a distal end of a catheter in contact with a trench in a circumference of a pulmonary vein, in accordance with an embodiment of the present invention;
FIG. 4 is a flow chart that schematically illustrates a method for ablation around the circumference of a pulmonary vein, in accordance with an embodiment of the present invention; and
FIGS. 5 - 7 are schematic pictorial illustrations and top views of distal ends of a catheter, in accordance with other embodiments of the present invention.

### DETAILED DESCRIPTION OF EMBODIMENTS

### OVERVIEW

Circumferential ablation procedures, such as pulmonary vein (PV) isolation, may be used for treating atrial fibrillation (AF). Such procedures typically ablate multiple points on the circumference of the PV ostium using, for example, a single-electrode ("point-by-point") catheter that ablates one point at a time. During PV isolation, a cardiologist creates lesions around an ostium of one or more pulmonary veins, so as to electrically isolate the musculature of the PVs.

During circumferential ablation, the point-by-point catheter may unintentionally enter the PV or slip along the wall of the atrium or a ridge. As a result, the catheter may accidentally ablate in a wrong position and may cause damage to the heart tissue. Furthermore, the cardiologist has to reposition the catheter against the planned ablation point. The catheter repositioning encumbers and prolongs the procedure and reduces the efficiency of the operating room (OR). In order to perform the ablation safely, the cardiologist has to navigate carefully along the circumference of the ostium so that the ablation catheter is positioned only in the intended ablation locations.

Embodiments of the present invention that are described herein below provide improved techniques for safe and accurate circumferential ablation. In some embodiments, an expandable distal-end device is coupled to the distal end of a catheter. The expandable distal-end device (e.g., an inflatable cork or a balloon) is configured to expand in a blood vessel and, when expanded, to form an annular guiding trench between the distal-end device and the inner circumference of the blood vessel. The annular trench formed between the expanded distal-end device and the blood vessel is sized and shaped to guide a medical instrument, such as a tip of a point-by-point ablation catheter, around the inner circumference of the blood vessel to the desired ablation points.

Consider, for example, a PV isolation procedure in accordance with an embodiment of the present invention. In such embodiments, the distal-end device is expanded at the ostium of the PV and forms an annular guiding trench around the ostium. The physician then guides the tip of an ablation catheter in the trench and performs ablation at multiple points around the ostium. The expanded distal-end device prevents the ablation catheter from entering too deeply into the PV and from slipping along the wall of the atrium, and also helps to maintain adequate physical contact between the ablation catheter and the tissue.

In some embodiments, the catheter is configured to inflate the distal-end device using a saline solution. The saline solution may comprise dye material that is visible in a fluoroscopic imaging system. In other embodiments, the distal-end device may comprise material opaque to X-ray radiation used in the fluoroscopic imaging system. These techniques improve detection of the distal-end device with respect to the PV.

The width of the atrium is typically larger than the diameter of the PV, and therefore the ostium of the PV has a funnel-shape. In an embodiment, the expanded distal-end device may have a conical shape that is configured to fit in the ostium and to form the annular guiding trench. When the distal-end device is expanded around the ostium, it may undesirably block pulmonary blood flow from the PV into the respective atrium. Typically, there is more than one PV per atrium so that the pulmonary blood may flow through another PV that remains open. Yet, in some embodiments the distal-end device may comprise one or more hollow tubes or other openings, through which the pulmonary blood may flow into the atrium while the distal-end device is fitted in the PV.

The disclosed techniques can be used with various structures of corks or balloons. For example, a hexagonal-shaped distal-end device (or any other suitable shape) may be used to form a trench that fits the shape of the ablation tip so as to hedge the tip during the circumferential ablation. Furthermore, the disclosed techniques may employ any device that fits into a catheter and can be extended in the ostium of a blood vessel. For example, the distal-end device may comprise a foldable device using any suitable mechanical, electrical or magnetic expansion mechanism.

The disclosed techniques prevent accidental ablation at unintended locations, and may shorten navigation time of the catheter to the next ablation point so as to reduce the overall duration of the PV isolation procedure. Moreover, the disclosed techniques are not limited to PV isolation procedures but are applicable to any circumferential diagnostic or therapeutic procedure. For example, the disclosed techniques may be used for isolating a superior vena cava (SVC), an inferior vena cava (IVC) or a left atrium appendage (LAA).

### SYSTEM DESCRIPTION

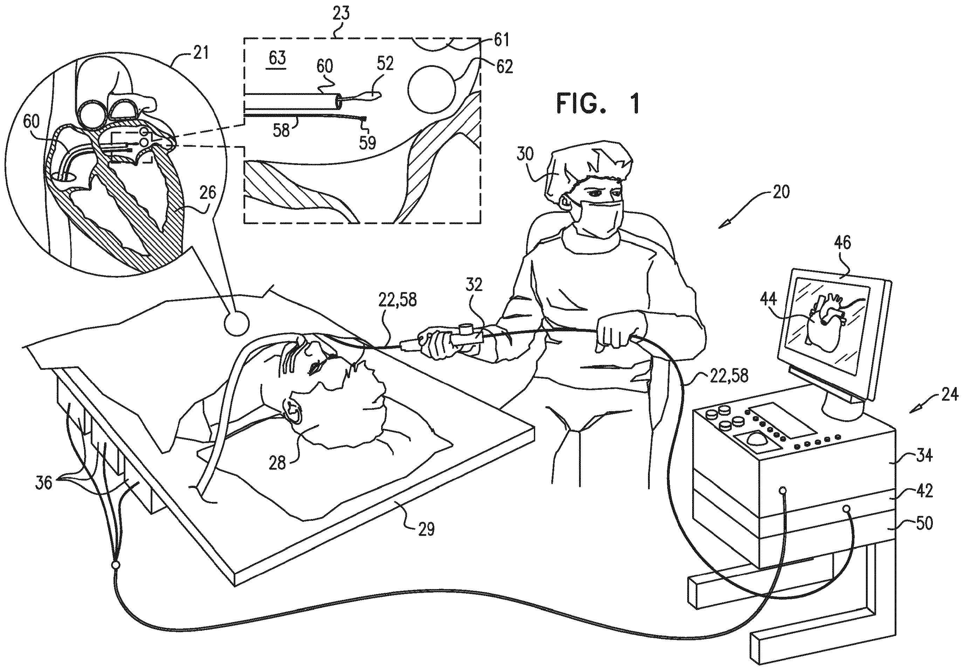

Fig. 1 is a schematic, pictorial illustration of a catheter-based tracking and ablation system 20, in accordance with an embodiment of the present invention. System 20 comprises an insertion tube 60, a cardiac catheter 22, an ablation catheter 58 and a control console 24. In the embodiment described herein, catheter 58 is used for ablation of tissue in a heart 26 in a PV isolation procedure, and catheter 22 is used for guiding the tip of catheter 58 to the desired ablation points.

Console 24 comprises a processor 42, typically a general-purpose computer, with suitable front end and interface circuits for receiving signals from catheter 22 and for controlling the other components of system 20 described herein.

A cardiologist 30 inserts catheter 22, using insertion tube 60, and catheter 58 directly through the vascular system of a patient 28. In some embodiments, cardiologist 30 inserts catheters 22 and 58 to cavity 63 separately so as to allow high maneuverability of catheter 58 during the ablation procedure. In alternative embodiments, cardiologist 30 may insert catheters 22 and 58 to cavity 63 together so as to reduce the amount of punctures in a septal wall of cavity 63 to a single puncture. The distal end of catheter 22 is connected to an expandable distal-end device, in the present example an inflatable balloon denoted cork 52.

A cavity 63 (e.g., left atrium) in heart 26 comprises pulmonary veins (PVs) 61 and 62. Cardiologist 30 navigates catheter 22 and catheter 58 to the vicinity of PV 62, as shown in an inset 21, using a manipulator 32 near the proximal end of the catheters. The proximal ends of catheter 22 and catheter 58 are connected to interface circuitry (not shown) comprised in processor 42.

Referring to an inset 23, during navigation of catheter 22, cork 52 is in a collapsed position so as to allow easy insertion and navigation of distal end 40 toward PV 62.

The positions of the distal ends of catheters 22 and 58 in cavity 63 are typically measured by applying magnetic position sensing techniques in catheter tracking system 20. In an embodiment, console 24 comprises a driver circuit 34, which drives magnetic field generators 36 placed at known positions external to patient 28 lying on table 29, e.g., below the patient's torso. The distal ends of each of the two catheters further comprises one or more magnetic field or impedance sensors, such as advanced current localization (ACL) sensors. The position sensors generate position signals in response to the sensed external magnetic fields, thereby enabling processor 42 to map the positions of the distal ends within the heart cavity. The magnetic position sensors are connected to the interface circuitry in processor 42. Cardiologist 30 can view the positions of the distal ends on an image 44 of heart 26 on a user display 46.

Processor 42 may be programmed in software to carry out the functions that are used by the system, and the processor stores data for the software in a memory 50. The software may be downloaded to console 24 in electronic form, over a network, for example, or it may be provided on non-transitory tangible media, such as optical, magnetic or electronic memory media. Alternatively, some or all of the functions of processor 42 may be carried out by dedicated or programmable digital hardware components.

This method of position sensing is implemented, for example, in the CARTO™ system, produced by Biosense Webster Inc. (Diamond Bar, Calif.) and is described in detail in U.S. Patents 5,391,199, 6,690,963, 6,484,118, 6,239,724, 6,618,612, 6,332,089, 6,690,963, 7,729,742 and 8,456,182, in PCT Patent Publication WO 96/05768, and in U.S. Patent Application Publication 2004/0068178 A1 and in U.S. Patent Application Publication 2015/0141798 A1, whose disclosures are all incorporated herein by reference.

### USING EXPANDABLE DISTAL-END DEVICE FOR PULMONARY VEIN ISOLATION

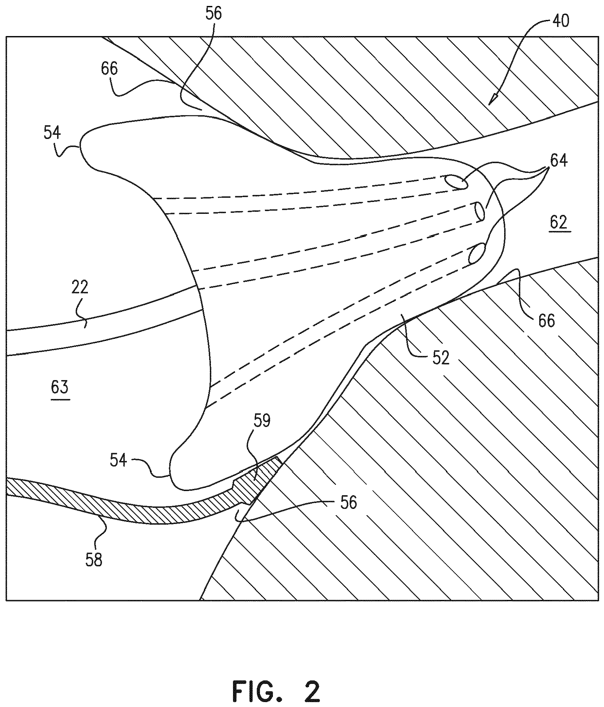

Fig. 2 is a schematic side view of a distal end 40 of catheter 22, and a distal tip 59 of catheter 58, at an ostium of pulmonary vein (PV) 62, in accordance with an embodiment of the present invention. During insertion of catheter 22, cork 52 is in a collapsed position. After insertion and navigation to a position of interest (e.g., the ostium of PV 62), cork 52 is inflated to an expanded position, typically using a bio-compatible saline liquid supplied by system 20. The figure shows cork 52 in its expanded position, after it has been fitted in the ostium of PV 62 and then inflated.

The diameter of PV 62 is typically smaller than the width of cavity 63 (e.g., the left atrium) of heart 26, and therefore the ostium of PV 62 comprises a funnel-shape. In some embodiments, as inflated, cork 52 assumes a conical shape that fits against the funnel shape of the ostium so that the external surface of cork 52 is brought into contact with a wall 66 in a circumference of PV 62, as shown in Fig. 2.

In some embodiments, cork 52 may comprise one or more openings, e.g., hollow tubes 64, which allow continuous flow of pulmonary blood from the patient's lungs (not shown) to heart 26, without causing formation of thrombus, while cork 52 is in the expanded position. In yet other embodiments, cork 52 may have a donut shape with a hollow center so as to allow blood flow from the lungs to PV 62 while cork 52 is in the expanded position. Cavity 63 typically comprises two PVs, e.g., PVs 61 (shown in Fig. 1) and 62. In alternative embodiments, cork 52 does not comprise tubes 64, and therefore completely blocks the ostium of PV 62. In this case pulmonary blood may flow from the respective lung to cavity 63, solely through PV 61.

In an embodiment, cork 52 comprises a protrusion 54 located at the perimeter of the wider end of cork 52. The protrusion is shaped to form an annular guiding trench 56 between cork 52 and wall 66 of PV 62. Trench 56 is shaped so that tip 59 of ablation catheter 58 fits into the trench without becoming captured between wall 66 and cork 52 during the PV isolation procedure. Cork 52 is further configured to maintain its stability even in an unlikely event of tip 59 being momentarily captured between wall 66 and cork 52. Tip 59 typically comprises one or more ablation electrodes for ablating tissue on wall 66.

The example configuration shown in Fig. 2 is chosen purely for the sake of conceptual clarity. In alternative embodiments, the disclosed techniques may use other shapes of balloons or corks, such as a hexagonal shaped cork or any other suitable shape that enables the formation of trench 56 having a desirable shape. The trench between cork 52 and wall 66 is configured to hedge tip 59 from entering PV 62 or slipping along the wall of cavity 63, away from the ostium of PV 62.

In an embodiment, the guiding trench sets the position of ablation tip 59 at the ostium of PV 62, thus reducing the probability of accidental ablation to unintended tissue, for example, due to navigation errors of the ablation catheter. For example, cork 52 may block catheter 58 from entering, by mistake, into PV 62 and ablating at a wrong position along PV 62. In addition, the guiding trench allows cardiologist 30 to perform accurate ablation around the ostium of PV 62 without unintended deviation of catheter 58 into cavity 63.

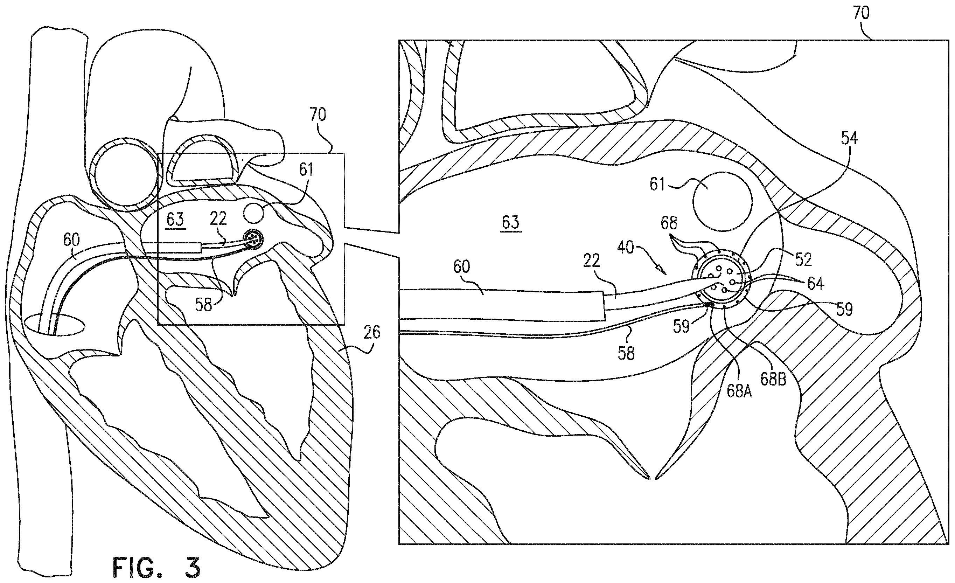

In other embodiments, cork 52 is fabricated from materials, such as nylon, PET, urethane or their variants, that are durable against high ablation power and do not conduct ablation power from tip 59 to undesired locations. By using such materials, the ablation power is applied only to intended ablation locations (e.g., ablation points 68 shown in Fig. 3 below).

During a PV isolation procedure, the cardiologist may use imaging techniques, such as fluoroscopy, for accurate navigation of cork 52 to the ostium of PV 62. In some embodiments, system 20 is configured to inject into cork 52 a dye saline, such as water-soluble iodine, which is visible to the fluoroscopic imaging system, so as to visualize the shape of the ostium of PV 62 while cork 52 is fitted into PV 62. In other embodiments, cork 52 may comprise a material that is opaque to fluoroscopic radiation (thus, appears dark in a fluoroscopic image) so as to further assist in the visualization of the ostium while cork 52 is fitted into PV 62.

The disclosed techniques may employ any device coupled to catheter 22 that is sufficiently small to enable insertion and navigation of the catheter, and can be extended in the ostium of PV 62. During insertion of the catheter, the device is in a folded position. After the distal end is positioned at the ostium of PV 62, the device is unfolded to an extended position, using any suitable unfolding mechanism (e.g., mechanical, electrical, magnetic) controlled by proximal end 32.

For example, the extendable device may comprise a foldable umbrella-shaped mechanism coupled to the distal end. The umbrella may be extended so that its edges extend out of PV 62, thus forming the annular guiding trench. In some embodiments, the apex of the umbrella is positioned in the ostium, pointing into PV 62, thus, the trench is formed below the extension and above wall 66. In alternative embodiments, the apex of the umbrella-shaped distal end points towards the left atrium. The "handle" of the umbrella is positioned in the ostium so that in unfolded position, the extension can be anchored to wall 66 and the trench is formed at the boundary of the extension.

FIG. 3 is a schematic, pictorial illustration of distal end 40 in contact with trench 56 in the circumference of PV 62, in accordance with an embodiment of the present invention. Cardiac catheter 22 and ablation catheter 58 are navigated by cardiologist 30 into cavity 63 (e.g., right atrium).

Referring to an inset 70, cork 52 is positioned in the ostium of PV 62 (not shown) and inflated using the saline delivered from proximal end 32 through tube 60. Hollow tubes 64 allow blood flow through cork 52, thereby improving hemodynamics. Furthermore, tubes 64 reduce a force required to counter the blood flow so as to maintain the position of cork 52, yet, tubes 64 may be redundant in case PV 61 is open. Protrusion 54 in the outer perimeter of cork 52 is configured to form annular guiding trench 56 in the circumference of cork 52. Trench 56 is shaped so that tip 59 snugly fits in to contact wall 66, as shown in Fig. 2.

Ablation catheter 58 is navigated to a selected ablation point 68A (one of ablation points 68) so as to perform a first lesion (typically 4 to 6 mm diameter). Tip 59 is further guided along trench 56 so as to ablate points 68 sequentially by attaching tip 59 to wall 66, at each intended ablation point (e.g., point 68A), and energizing catheter 58 using RF electrical current. The guiding trench is configured to secure accurate positioning of tip 59 relative to wall 66, thus, preventing accidental ablation at wrong locations (e.g., deeply into PV 62). Furthermore, trench 56 may assist in shortening the navigation time of tip 59 to an ablation point 68B, which is the next ablation point in the sequence. The diameter of each lesion point 68 (dictated by RF power and ablation time) and the gaps between adjacent points 68 are predefined by cardiologist 30 so as to obtain a complete isolation of the respective PV (e.g., PV 62).

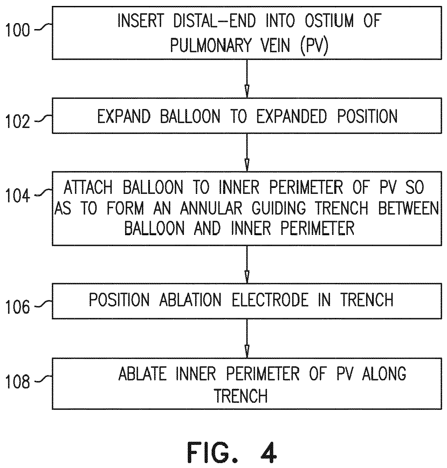

Fig. 4 is a flow chart that schematically illustrates a method for ablating wall 66 in the circumference of the ostium of PV 62, in accordance with an embodiment of the present invention. The method begins at an insertion step 100, in which cardiologist 30 uses insertion tube 60 to insert catheter 22 into cavity 63 while ablation catheter 58 is typically inserted separately. Cardiologist 30 navigates cork 52, which is coupled to distal end 40 of catheter 22, into the ostium of PV 62. At an expansion step 102, cardiologist 30 inflates cork 52 using saline solution (or any other suitable inflating technique) so as to assume a conical shape of cork 52 that fits against the funnel shape of the ostium of PV 62. At a trench formation step 104, cardiologist 30 attaches cork 52 to the inner circumference of PV 62 so as to form an annular guiding trench 56 between cork 52 and wall 66 of PV 62. As cork 52 is properly attached to PV62, cardiologist 30 is free to perform the ablation procedure, rather than being occupied with holding catheter 22 against the flow of the pulmonary blood.

At a positioning step 106, cardiologist positions tip 59 of ablation catheter 58 within trench 56 attached to wall 66 at ablation point 68A. The shape of trench 56 is designed to guide tip 59 within the trench so as to prevent the tip from slipping away from point 68A. At an ablation step 108, cardiologist 30 energizes ablation catheter 58 using RF electrical current so as to ablate point 68A. After concluding the ablation of point 68A, cardiologist 30 may guide tip 59 along trench 56 to an adjacent ablation point 68B. The cardiologist then repeats the ablation of wall 66 at point 68B, and repeats the described sequence until concluding the ablation of all points 68.

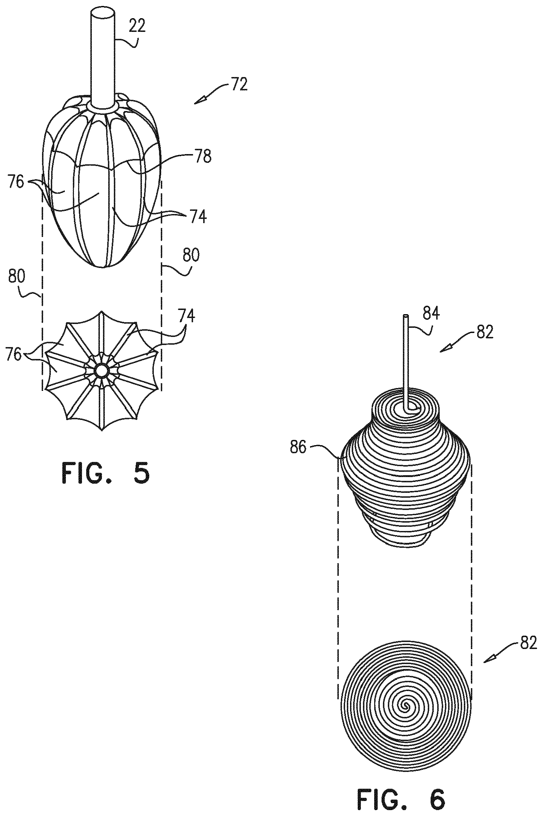

Fig. 5 is a schematic pictorial illustration and a top view of a distal end 72 of catheter 22, in accordance with another embodiment of the present invention. Distal end 72 may, for example, replace cork 52 at distal end 40 of Fig. 2 above.

Distal end 72 comprises an inflatable balloon 76, which is configured to expand into an expandable position, and arms 74 (also denoted splines), which are configured to form a desired shape of balloon 76 in the expanded position (as shown in the top view).

During the insertion of catheter 22, balloon 76 is initially held in a collapsed position. After navigation of the distal end to the ostium of PV 62 (shown in Fig. 2), balloon 76 is inflated to the expanded position, using the technique described in Fig. 2 above. The schematic illustration in Fig. 5 shows balloon 76 in its expanded position, after being fitted against the ostium of PV 62.

In an embodiment, arms 74 that are coupled to balloon 76, are typically made of bio-compatible materials (e.g., nitinol or pre-shaped materials of cork 52, such as nylon, PET, or urethane). The arms are configured to shape balloon 76 in the expanded position as shown in the top view. Arms 74 may be coupled to balloon 76 in a longitude arrangement, as shown in Fig. 5, or in any other suitable arrangement. A line 78 represents the largest dimension of distal end 72 in the expanded position. This dimension determines the maximal outer perimeter of distal end 72 as shown by dashed lines 80.

In some embodiments, when expanded, distal end 72 assumes a polygon shape as dictated by arms 74 and shown in the top view. Alternatively, the distal end may assume any other suitable shape that provides the best fit against the ostium of PV 62.

In other embodiments, balloon 76 may comprise one or more openings, such as hollow tubes 64, which allow continuous flow of pulmonary blood from the patient's lungs (not shown) to heart 26 as described in Fig. 2 above.

In yet other embodiments, balloon 76 may comprise irrigation tubes (similar to tubes 64 shown in Fig. 2) for the saline that apply irrigation while balloon 76 still expanded to maintain the required pressure against PV 62.

Fig. 6 is a schematic pictorial illustration and a top view of a distal end 82 of catheter 22 in accordance with yet another embodiment of the present invention. Distal end 82 may provide the same functionality as distal ends 40 and 72, however distal end 82 comprises a pre-formed spring made of a wire 84 rather than an inflatable balloon. Wire 84 is typically fabricated from bio-compatible nitinol or any other suitable material, and its top vertex is comprised in catheter 22.

In some embodiments, during the insertion and navigation phases, wire 84 may be forced into a sheath (not shown) in a collapsed position. When distal end 82 is positioned at the desired location (e.g., in front of ostium of PV 62), wire 84 may be forced out of the sheath so as to expand to its pre-formed profile as shown in Fig. 6.

The external perimeter of the spring may have any desired shape, such as a round shape, as shown in the top view of Fig. 6. Position 86 represents the largest dimension of distal end 82. This dimension determines the outer perimeter of distal end 82 as shown by dashed lines 82.

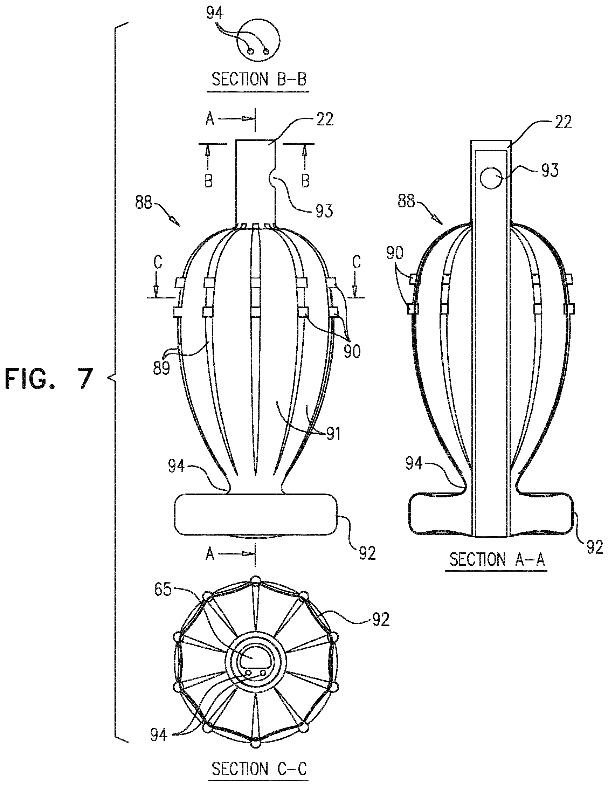

Fig. 7 is a schematic pictorial illustration and sectional views B-B and C-C of a distal end 88 of catheter 22, in accordance with another embodiment of the present invention. Distal end 88 may, for example, replace cork 52 at distal end 40 of Fig. 2 above.

Distal end 88 comprises an inflatable balloon 91, which is substantially similar to balloon 76 shown in Fig. 5 and is configured to form trench 56 as depicted in Fig. 2. Balloon 91 is configured to expand into an expandable position, and arms 89 (similar to arms 74 in Fig. 5) are configured to form a desired shape of balloon 91 in the expanded position (e.g., as shown in section C-C). Distal end 88 further comprises electrodes 90, such as electrophysiological (EP) mapping electrodes, coupled to arms 89 and configured to sense electrical potentials from PV 62 and generate respective potential gradient signals. In an embodiment, electrodes 90 may be formed on a printed circuit board (PCB) and attached to arms 89. In alternative embodiment, arms 89, including electrodes 90, are produced on a flexible PCB.

The gradient signals are transmitted to processor 42 via wires (not shown) comprised in catheter 22. In other embodiments, electrodes 90 may comprise other types of electrodes such as position sensors, contact sensors (e.g., to monitor whether catheter 58 is in contact with balloon 91), temperature sensors (e.g., to monitor formation of a lesion), ablation electrodes or any other type of electrode or sensor, suitable for performing a respective medical procedure.

Distal end 88 further comprises an anchor 92, connected to balloon 91 via a neck section 94. Anchor 92 is also an inflatable balloon. In an inflatable position, the balloon anchors distal end 88 to a desirable position along PV 62 so as to allow cardiologist 30 to perform the ablation procedure, and frees the cardiologist from holding catheter 22 against the flow of the pulmonary blood.

During the insertion of catheter 22, balloon 91 and anchor 92 are initially held in a collapsed position. After navigation of the distal end to the ostium of PV 62 (shown in Fig. 2), balloon 91 and anchor 92 are inflated to the expanded position, using a technique similar to the technique described in Fig. 2 above. The schematic illustration in Fig. 7 shows balloon 91 and anchor 92 in their expanded position, after being fitted against the ostium of PV 62. In alternative embodiments, anchor 92 may be a spring, made of a wire (such as wire 84), as described in Fig. 6 above.

In some embodiments, when expanded, distal end 88 assumes a polygon shape as shown in section C-C. Alternatively, the distal end may assume any other suitable shape to fit against the ostium of PV 62.

In other embodiments, balloon 91 may comprise a hollow tube 65 (similar to tubes 64 depicted in Fig. 2), which allows a continuous flow of pulmonary blood from the patient's lungs (not shown) to heart 26, as described in Fig. 2 above. In addition, catheter 22 comprises an opening 93, through which the pulmonary blood may flow out of tube 65 on its route to heart 26. In alternative embodiments, opening 93 may be formed as an exit of channels (such as tubes 64 shown in Fig. 2) comprised in balloon 91. Such opening 93 may be located, for example, above electrodes 90, as a single or multiple openings.

In yet other embodiments, distal end 88 comprises channels 94 used for inflating (and deflating) balloon 91 and anchor 92. The number of channels may be two, as shown in Fig. 7, or any other suitable number of channels. In an embodiment, balloon 91 and anchor 92 are inflated independently using two separate channels, in which case catheter 22 comprises at least two channels 94. In an alternative embodiment, balloon 91 and anchor 92 may each have, an internal valve (not shown) so that a single channel may be sufficient to inflate both the balloon and anchor. In another embodiment, at least one of channels 94 may be used for wiring electrodes 90 to processor 42.

In yet another embodiment, at least one of channels 94 may be used for irrigating tube 65 for various purposes, such as for flushing out blood residues from neck 94.

The distal end configurations shown in Figs. 2, 5, 6 and 7 are example configurations that are depicted purely for the sake of conceptual clarity. In alternative embodiments, any other suitable distal end configuration can be used.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

Aspects of the invention:
1. A method, comprising:
   inserting into a patient body a catheter, which comprises an insertion tube and at least an expandable distal-end device coupled to a distal end of the insertion tube;
   expanding the distal-end device in a blood vessel, thereby forming an annular guiding trench between the distal-end device and a circumference of the blood vessel, the annular trench shaped to receive and guide a distal tip of a medical instrument; and
   guiding the distal tip of the medical instrument in the annular guiding trench formed by the expanded distal-end device of the catheter, so as to approach multiple points on the circumference of the blood vessel.
2. The method according to aspect 1, wherein expanding the distal-end device comprises inflating the distal-end device using a saline solution.
3. The method according to aspect 2, wherein the saline solution comprises dye visible to X-ray radiation.
4. The method according to aspect 1, wherein the distal-end device comprises a material that is opaque to X-ray radiation.
5. The method according to aspect 1, wherein the blood vessel comprises a pulmonary vein (PV).
6. The method according to aspect 1, wherein expanding the distal-end device comprises inflating the distal-end device so as to form a shape that fits a circumference of an ostium of the blood vessel.
7. The method according to aspect 1, wherein the distal-end device comprises at least one opening, which is configured to allow blood flow through the distal-end device when the distal-end device is expanded in the blood vessel.
8. The method according to aspect 1, wherein the distal-end device comprises a protrusion, and wherein the annular guiding trench is formed between the protrusion and the circumference of the blood vessel.
9. The method according to aspect 1, wherein the distal tip of the medical instrument comprises an ablation electrode, and wherein guiding the distal tip comprises ablating multiple points around the circumference of the blood vessel using the ablation electrode.
10. The method according to aspect 1, wherein the distal-end device comprises multiple foldable arms, and wherein expanding the distal-end device comprises unfolding the arms so as to form the annular trench.
11. The method according to aspect 1, wherein the distal-end device comprises an expandable spring, and wherein expanding the distal-end device comprises expanding the expandable spring so as to form the annular trench.
12. The method according to aspect 1, wherein expanding the distal-end device comprises expanding an anchoring extension in the blood vessel, thereby fixing the distal-end device to the circumference of the blood vessel.

## Claims

1. A catheter, comprising:
an insertion tube for insertion into a patient body; and
at least an expandable distal-end device coupled to a distal end of the insertion tube, wherein the expandable device is configured to expand the blood vessel thereby forming an annular guiding trench between the distal-end device and a circumference of the blood vessel, the annular trench shaped to receive and guide a distal tip of a medical instrument.

2. The catheter according to claim 1, wherein the distal-end device is configured to inflate using a saline solution.

3. The catheter according to claim 2, wherein the saline solution comprises dye visible to X-ray radiation.

4. The catheter according to claim 1, wherein the distal-end device comprises a material that is opaque to X-ray radiation.

5. The catheter according to claim 1, wherein the blood vessel comprises pulmonary vein (PV).

6. The catheter according to claim 1, wherein, at an expanded position, the distal-end device is configured to form a shape that fits a circumference of an ostium of the blood vessel.

7. The catheter according to claim 1, wherein the distal-end device comprises at least one opening, which is configured to allow blood flow through the distal-end device when the distal-end device is expanded in the blood vessel.

8. The catheter according to claim 1, wherein the distal-end device comprises a protrusion, and wherein the annular guiding trench is formed between the protrusion and the circumference of the blood vessel.

9. The catheter according to claim 1, wherein the distal tip of the medical instrument comprises an ablation electrode, which is configured to ablate multiple points around the circumference of the blood vessel using the ablation electrode.

10. The catheter according to claim 1, wherein the distal-end device comprises multiple foldable arms, and is configured to unfold the arms so as to form the annular trench.

11. The catheter according to claim 1, wherein the distal-end device comprises an expandable spring, which is configured to expand so as to form the annular trench.

12. The catheter according to claim 1, wherein the distal-end device comprises an anchoring extension, which is configured to expand in the blood vessel thereby fixing the distal-end device to the circumference of the blood vessel.
